# NEW EUROPEAN PATENT SPECIFICATION

(11) **EP 1 005 867 B2**
(45) Date of publication and mention of the opposition decision: **17.02.2010**
(45) Mention of the grant of the patent: 03.12.2003
(21) Application number: 99203920.6
(22) Date of filing: 16.12.1994
(51) Int. Cl.: A61K 38/16, A61P 1/04, A61P 43/00

(54) **Botulinum toxins for modulating cholinergic controlled secretions**
Botulinustoxine zur Modulation cholinergisch-kontrollierter Sekretionen
Toxines de botulinum pour la modulation des secretions controlées par le système cholinergique

(30) Priority: 28.12.1993 US 173996
(43) Date of publication of application: 07.06.2000
(62) Divisional of application: 95906674.7
(73) Proprietor: ALLERGAN, INC., Irvine CA 92612 (US)
(72) Inventor: Aoki, Roger K., Laguna Hills, CA 92653 (US); Grayston, Michael W., Irvine, CA 92714 (US); Carlson, Steven R., Laguna Niguel, CA 92677 (US); Leon, Judith M., Laguna Niguel, CA 92677 (US)
(74) Representative: HOFFMANN EITLE

(56) References cited:
- WO-A-95/28171
- KONDO ET AL.: "Modification of the action of pentagastrin on acid secretion by botulinum toxin" EXPERIENTIA, vol. 33, 1977, pages 750-751, XP000994885
- JANKOVIC J ET AL: "THERAPEUTIC USES OF BOTULINUM TOXIN" NEW ENGLAND JOURNAL OF MEDICINE, THE,US,MASSACHUSETTS MEDICAL SOCIETY, WALTHAM, MA, vol. 324, no. 17, 25 April 1991 (1991-04-25), pages 1186-1194, XP000650928 ISSN: 0028-4793
- RAY ET AL.: "Botulinum toxin inhibits arachidonic acid release associated with acetylcholine release from PC12 cells" J. BIOL. CHEM., vol. 268, no. 15, 1993, pages 11057-11064, XP000986197
- BRODTKORB ET AL.: 'Transdermal scopolamine in drooling' J. OF MENTAL DEFICIENCY RESEARCH vol. 32, 1988, pages 233 - 237
- ADENIS ET AL.: 'Traitement des spasmes faciaux par la toxine botulique A' J. FR. OPHTALMOL. vol. 13, no. 5, 1990, pages 259 - 264

## Description

### FIELD OF THE INVENTION

The invention provides the use of botulinum toxin for the manufacture of a medicament for the reduction of cholinergic controlled or cholinergic influenced secretions, wherein the secretion is not lacrimation.

### BACKGROUND OF THE INVENTION

Heretofore, Botulinum toxins, in particular Botulinum toxin type A, has been used in the treatment of a number of neuromuscular disorders and conditions involving muscular spasm; for example, strabismus, blepharospasm, spasmodic torticollis (cervical dystonia), oromandibular dystonia and spasmodic dysphonia (laryngeal dystonia) (See e.g. Jankovic et al. New England Journal of Medicine (1991)1186). The toxin binds rapidly and strongly to presynaptic cholinergic nerve terminals and inhibits the exocytosis of acetylcholine by decreasing the frequency of acetylcholine release. This results in local paralysis and hence relaxation of the muscle afflicted by spasm.

Kondo et al in Specialia (1977) 751 report a modification of the action of pentagastrin on acid secretion by botulinum toxin. Ray et al. in J. Biol. Chem (1993) 11057 describe the inhibition of arachidonic acid release associated with acetylcholine release from PC12 cells with botulinum toxin.

For one example of treating neuromuscular disorders, see U.S. Patent No. 5,053,005 to Borodic, which suggests treating curvature of the juvenile spine, i.e., scoliosis, with an acetylcholine release inhibitor, preferably Botulinum toxin A.

For the treatment of strabismus with Botulinum toxin type A, see Elston, J.S., et al., British Journal of Ophthalmology, 1985, 69, 718-724 and 891-896. For the treatment of blepharospasm with Botulinum toxin type A, see Adenis, J.P., et al., J. Fr. Ophthalmol., 1990, 13 (5) at pages 259-264. For treating squint, see Elston, J.S., Eye, 1990, 4(4):VII. For treating spasmodic and oromandibular dystonia torticollis, see Jankovic et al., Neurology, 1987, 37, 616-623.

Spasmodic dysphonia has been treated with Botulinum toxin type A. See Blitzer et al., Ann. Otol. Rhino. Laryngol, 1985, 94, 591-594. Lingual dystonia was treated with Botulinum toxin type A according to Brin et al., Adv. Neurol. (1987) 50, 599-608. Finally, Cohen et al., Neurology (1987) 37 (Suppl. 1), 123-4, discloses the treatment of writer's cramp with Botulinum toxin type A.

The term Botulinum toxin is a generic term embracing the family of toxins produced by the anaerobic bacterium *Clostridium botulinum and, to date,* seven immunologically distinct neurotoxins have been identified. These have been given the designations A, B, C, D, E, F and G. For further information concerning the properties of the various Botulinum toxins, reference is made to the article by Jankovic and Brin, The New England Journal of Medicine, No. 17, 1990, pp. 1186-1194, and to the review by Charles L. Hatheway in Chapter 1 of the book entitled Botulinum Neurotoxin and Tetanus Toxin, L. L. Simpson, Ed., published by Academic Press Inc. of San Diego, California, 1989, the disclosures in which are incorporated herein by reference.

The neurotoxic component of Botulinum toxin has a molecular weight of about 150 kilodaltons and is thought to comprise a short polypeptide chain of about 50 kD which is considered to be responsible for the toxic properties of the toxin, i.e., by interfering with the exocytosis of acetylcholine, by decreasing the frequency of acetylcholine release, and a larger polypeptide chain of about 100 kD which is believed to be necessary to enable the toxin to bind to the presynaptic membrane.

The "short" and "long" chains are linked together by means of a simple disulfide bridge. (It is noted that certain serotypes of Botulinum toxin, e.g., type E, may exist in the form of a single chain un-nicked protein, as opposed to a dichain. The single chain form is less active but may be converted to the corresponding dichain by nicking with a protease, e.g., trypsin. Both the single and the dichain are useful in the method of the present invention.)

In general, four physiologic groups of C. botulinum are recognized (I, II, III, IV). The organisms capable of producing a serologically distinct toxin may come from more than one physiological group. For example, Type B and F toxins can be produced by strains from Group I or II. In addition, other strains of clostridial species (*C. baratii*, type F; *C*. *butyricum*, type E; *C. novyi*, type C₁ or D) have been identified which can produce botulinum neurotoxins.

Immunotoxin conjugates of ricin and antibodies, which are characterized as having enhanced cytotoxicity through improving cell surface affinity, are disclosed in European Patent Specification 0 129 434. The inventors note that botulinum toxin may be utilized in place of ricin.

Botulinum toxin is obtained commercially by establishing and growing cultures of *C. botulinum* in a fermenter and then harvesting and purifying the fermented mixture in accordance with known techniques.

Botulinum toxin type A, the toxin type generally utilized in treating neuromuscular conditions, is currently available commercially from several sources; for example, from Porton Products Ltd. UK, under the trade name "DYSPORT," and from Allergan, Inc. , Irvine, California, under the trade name BOTOX®.

### SUMMARY OF THE INVENTION

The present invention relates to the use of botulinum toxin for the manufacture of a medicament as set forth in the claims. The Botulinum toxin is selected from the group consisting of Botulinum toxin type A, B, C, D, E, F and G.

Each serotype of Botulinum toxin has been identified as immunologically different proteins through the use of specific antibodies. For example, if the antibody (antitoxin) recognizes, that is, neutralizes the biological activity of, for example, type A it will not recognize types B,C,D, E, F or G.

While all of the Botulinum toxins appear to be zinc endopeptidases, the mechanism of action of different serotypes, for example, A and E within the neuron appear to be different than that of Type B. In addition, the neuronal surface "receptor" for the toxin appears to be different for the serotypes.

In the area of use of the Botulinum toxins in accordance with the present invention with regard to organ systems which involve the release of neurotransmitter, it is expected to introduce the toxins A, B, C, D, E, F, and G directly by local injections.

### DETAILED DESCRIPTION

The Botulinum toxins used according to the present invention are Botulinum toxins type A, B, C, D, E, F and G.

The physiologic groups of *Clostridium botulinum* types are listed in Table I.

**Table 1**

| Physiologic Groups of *Clostridium botulinum* | | | | | | | |
|---|---|---|---|---|---|---|---|
| Group | Toxin Sero-Type | Biochemistry | Milk Digest | Gluose Fermentation | Lipase | Phages & Plasmids | Phenotypically Related Clostridium (nontoxigenic) |
| I | A,B,F | proteolytic saccharolytic | + | + | + | + | C. sporogenes |
| II | B,E,F | nonproteolytic saccharolytic psychotrophic | - | + | + | + | |
| III | C,D | nonproteolytic saccharolytic | ± | + | + | + | C. novyi |
| IV | G | proteolytic nonsaccharolytic | + | - | - | - | C. subterminale |

These toxin types may be produced by selection from the appropriate physiologic group of *Clostridium botulinum organisms*. The organisms designated as Group I are usually referred to as proteolytic and produce Botulinum toxins of types A, B and F. The organisms designated as Group II are saccharolytic and produce Botulinum toxins of types B, E and F. The organisms designated as Group III produce only Botulinum toxin types C and D and are distinguished from organisms of Groups I and II by the production of significant amounts of propionic acid. Group IV organisms only produce neurotoxin of type G. The production of any and all of the Botulinum toxin types A, B, C, D, E, F and G are described in Chapter 1 of *Botulinum Neurotoxin and Tetanus Toxin*, cited above, and/or the references cited therein. Botulinum toxins types B, C, D, E, F and G are also available from various species of clostridia.

Currently fourteen species of clostridia are considered pathogenic. Most of the pathogenic strains produce toxins which are responsible for the various pathological signs and symptoms. Organisms which produce Botulinum toxins have been isolated from botulism outbreaks in humans (types A, B, E and F) and animals (types C and D). Their identities were described through the use of specific antitoxins (antibodies) developed against the earlier toxins. Type G toxin was found in soil and has low toxigenicity. However, it has been isolated from autopsy specimens, but thus far there has not been adequate evidence that type G botulism has occurred in humans.

Preferably, the toxin is to be administered directly to the affected region. The toxin can be presented as a sterile pyrogen-free aqueous solution or dispersion and as a sterile powder for reconstitution into a sterile solution or dispersion.

Where desired, tonicity adjusting agents such as sodium chloride, glycerol and various sugars can be added. Stabilizers such as human serum albumin may also be included. The formulation may be preserved by means of a suitable pharmaceutically acceptable preservative such as a paraben, although preferably it is unpreserved.

It is preferred that the toxin is formulated in unit dosage form; for example, it can be provided as a sterile solution in a vial or as a vial or sachet containing a lyophilized powder for reconstituting a suitable vehicle such as saline for injection.

In one embodiment, the Botulinum toxin is formulated in a solution containing saline and pasteurized human serum albumin, which stabilizes the toxin and minimizes loss through non-specific adsorption. The solution is sterile filtered (0.2 micron filter), filled into individual vials and then vacuum-dried to give a sterile lyophilized powder. In use, the powder can be reconstituted, by the addition of sterile unpreserved normal saline (sodium chloride 0.9% for injection).

The dose of toxin to be administered to the patient will depend upon the severity of the condition, the age and size of the patient and the potency of the toxin. The potency of the toxin is expressed as a multiple of the LD₅₀ value for the mouse, one unit (U) of toxin being defined as being the equivalent amount of toxin that kills 50% of a group of 18 to 20 female Swiss-Webster mice, weighing about 20 grams each.

Typically, the dose to be administered to the patient may be up from about 0.01 to about 1,000 units; for example, up to about 500 units, and preferably in the range from about 80 to about 460 units per patient per treatment, although smaller or larger doses may be administered in appropriate circumstances such as up to about 50 units for controlling cholinergic secretions.

As the physicians become more familiar with the use of this product, the dose may be changed. In the Botulinum toxin type A, available from Porton, DYSPORT, 1 nanogram (ng) contains 40 units. 1 ng of the Botulinum toxin type A, available from Allergan, Inc., i.e., BOTOX®, contains 4 units. The potency of Botulinum toxin and its long duration of action mean that doses will tend to be administered on an infrequent basis. Ultimately, however, both the quantity of toxin administered and the frequency of its administration will be at the discretion of the physician responsible for the treatment and will be commensurate with questions of safety and the effects produced by the toxin.

The invention will now be illustrated by reference to the following nonlimiting example.

In the example, appropriate areas of each patient are injected with a sterile solution containing the confirmation of Botulinum toxin. Total patient doses range from about 0.01 units to 460 units. General anaesthesia, local anaesthesia and sedation are used according to the age of the patient, the number of sites to be injected, and the particular needs of the patient. More than one injection and/or sites of injection may be necessary to achieve the desired result.

Following injection, it is noted that there are no systemic or local side effects and none of the patients are found to develop extensive local hypotonicity. The majority of patients show an improvement in function both subjectively and when measured objectively.

### Example 1

### The Use of Botulinum toxin Types A-G in the Treatment of Excessive Sweating, or Mucus Secretion or Other Cholinergic Controlled Secretions

A male, age 65, with excessive unilateral sweating is treated by administering 0.01 to 50 units, of Botulinum toxin, depending upon degree of desired effect. The larger the dose, usually the greater spread and duration of effect. Small doses are used initially. Any serotype toxin alone or in combination could be used in this indication. The administration is to the gland nerve plexus, ganglion, spinal cord or central nervous system to be determined by the physician's knowledge of the anatomy and physiology of the target glands and secretary cells. In addition, the appropriate spinal cord level or brain area can be injected with the toxin (although this would cause many effects, including general weakness). Thus, the gland (if accessible) or the nerve plexus or ganglion are the targets of choice. Excessive sweating, mucus secretion or gastrointestinal secretions are positively influenced by the cholinergic nervous system. Sweating is under greater cholinergic control than mucus or gastric secretion and would respond better to toxin treatment. However, mucus and gastric secretions could be modulated through the cholinergic system. All symptoms would be reduced or eliminated with toxin therapy in about 1-7 days. Duration would be weeks to several months.

## Claims

1. The use of a botulinum toxin for the manufacture of a medicament for the reduction of a cholinergic controlled or cholinergic influenced secretion, wherein the secretion is not lacrimation.

2. The use of a botulinum toxin according to claim 1 for the manufacture of a medicament for reducing a mucus secretion.

3. The use according to claim 1 or claim 2 wherein the botulinum toxin is to be administered directly to an affected area of a patient.

4. The use according to any one of claims 1 to 3 wherein the botulinum toxin is to be administered by local administration to a gland.

5. The use according to claim 4 wherein the botulinum toxin type A is to be administered to a patient in an amount of between about 0.01 and about 50 units.

## Patentansprüche

1. Verwendung eines Botulinumtoxins zur Herstellung eines Medikamentes zur Verminderung einer cholinergisch gesteuerten oder cholinergisch beeinflussten Sekretion, worin die Sekretion keine Tränenbildung ist.

2. Verwendung eines Botulinumtoxins nach Anspruch 1, zur Herstellung eines Medikamentes zur Reduzierung einer Mukussekretion.

3. Verwendung nach Anspruch 1 oder 2, worin das Botulinumtoxin direkt auf das befallene Gebiet eines Patienten zu verabreichen ist.

4. Verwendung nach einem der Ansprüche 1 bis 3, worin das Botulinumtoxin durch lokale Verabreichung an eine Drüse zu verabreichen ist.

5. Verwendung nach Anspruch 4, worin das Botulinumtoxin vom Typ A an einen Patienten in einer Menge zwischen etwa 0,01 und etwa 50 Einheiten zu verabreichen ist.

## Revendications

1. Utilisation d'une toxine de botulinum pour la fabrication d'un médicament pour la diminution d'une sécrétion contrôlée ou influencée par le système cholinergique, dans laquelle la sécrétion n'est pas la lacrymation.

2. Utilisation d'une toxine de botulinum selon la revendication 1 pour la fabrication d'un médicament pour la diminution d'une sécrétion de mucus.

3. Utilisation selon la revendication 1 ou la revendication 2 dans laquelle la toxine de botulinum est destinée à être administrée directement à une zone affectée du patient.

4. Utilisation selon l'une quelconque des revendications 1 à 3 dans laquelle la toxine de botulinum est destinée à être administrée par voie locale à une glande.

5. Utilisation selon la revendication 4 dans laquelle la toxine de botulinum de type A est destinée à être administrée à un patient dans une quantité se situant dans la gamme d'environ 0,01 à environ 50 unités.
